# EUROPÄISCHE PATENTSCHRIFT

(11) **EP 1 562 092 B1**
(45) Veröffentlichungstag und Bekanntmachung des Hinweises auf die Patenterteilung: **21.07.2010**
(21) Anmeldenummer: 05001527.0
(22) Anmeldetag: 26.01.2005
(51) Int. Cl.: G05D 7/06, A61M 11/00, A61M 11/06, A61M 13/00, A61M 15/00

(54) **Dosiervorrichtung**
Dosing device
Appareil de dosage

(30) Priorität: 05.02.2004 DE 102004006450
(43) Veröffentlichungstag der Anmeldung: 10.08.2005
(73) Patentinhaber: Ing. Erich Pfeiffer GmbH, 78315 Radolfzell (DE)
(72) Erfinder: Körner, Joachim, 88690 Uhldingen (DE); Helmlinger, Michael, 78315 Radolfzell (DE); Schürle, Holger, 78315 Radolfzell (DE)
(74) Vertreter: Patentanwälte Ruff, Wilhelm, Beier, Dauster & Partner

(56) Entgegenhaltungen:
- WO-A-2005/025654
- US-A- 5 452 711
- US-A- 5 569 190
- US-A- 5 893 515

## Beschreibung

Die Erfindung betrifft eine Dosiervorrichtung mit einer Fördereinrichtung für wenigstens ein Medium, mit einem Mediumspeicher zur Bevorratung des Mediums und mit einem Applikator zur Abgabe des Mediums, mit einer Steuereinheit zur Beeinflussung der Fördereinrichtung und mit einem elektrischen Energiespeicher zur Versorgung der Steuereinheit und/oder der Fördereinrichtung.

Dabei sind die Fördereinrichtung, der Mediumspeicher und der Applikator einerseits und die Steuereinheit und der elektrische Energiespeicher andererseits in jeweils separaten Baueinheiten untergebracht. Weiterhin sind Führungsmittel zum Ineinanderstecken der Baueinheiten vorgesehen, die eine elektromechanische Schnittstelle für eine werkzeuglose An- und/oder Abkopplung relativ zu der Steuereinheit und/oder dem Energiespeicher aufweisen.

Eine derartige Dosiervorrichtung ist aus der US 5,452,711 bekannt. Die dort gezeigt Dosiervorrichtung besteht aus zwei aneinander ansteckbaren Baueinheit, die beim Anstecken auch elektrisch miteinander gekoppelt werden.

Bei einem weiteren bekannten Typ einer Dosiervorrichtung, wie er in der DE 2854841C2 beschrieben wird, ist ein Mediumspeicher vorgesehen, in dem das auszutragende Medium bis zu einem Austragvorgang gespeichert ist. Während des Austragvorgangs wird das Medium durch eine elektrisch angetriebene Fördereinrichtung aus dem Mediumspeicher gefördert und kann durch einen Applikator in eine Umgebung der Dosiervorrichtung abgegeben werden. Um eine präzise Dosierung des ausgetragenen Mediums sicherzustellen, ist eine Steuereinheit zur Beeinflussung der Fördereinrichtung vorgesehen, die einen Austrag des Mediums steuern kann. Zum Betrieb der Steuereinheit und der Fördereinrichtung ist ein elektrischer Energiespeicher an der Dosiervorrichtung vorgesehen.

Die Aufgabe der Erfindung besteht darin, eine Dosiervorrichtung der eingangs genannten Art in Hinblick auf die Kopplungs der Baueinheiten zu verbessern.

Diese Aufgabe wird dadurch gelöst, dass die eine Baueinheit mit einem zu einer Stirnseite offenen Aufnahmeraum versehen ist, in den ein korrespondierender Steckabschnitt der anderen Baueinheit einführbar ist, wobei der Steckabschnitt den Medienspeicher umfasst.

Durch eine Integration aller mediumführenden Komponenten wie der Fördereinrichtung, dem Mediumspeicher und dem Applikator in eine gemeinsame Baueinheit kann auf aufwendige Kupplungs- und/oder Abdichtmaßnahmen zwischen den mediumführenden Komponenten wie der Fördereinrichtung, dem Mediumspeicher und dem Applikator verzichtet werden. Mit der Integration der Steuereinheit und des Energiespeichers in eine zweite Baueinheit kann eine Anzahl elektrischer Kontakte und eine Leitungslänge elektrischer Verbindungen gering gehalten werden. Damit ist eine einfachere, zuverlässigere und kostengünstigere Ausführung der Dosiereinrichtung möglich. Die Führungsmittel können linear oder nichtlinear ausgeführt sein. Bevorzugt weisen die Führungsmittel Geradführungen oder Kurvenführungen mit oder ohne Steigung auf. Durch die Führungsmittel und durch die elektromechanische Schnittstelle kann in besonders einfacher Weise eine Verbindung zwischen den Baueinheiten hergestellt werden. Die Führungsmittel geben einem Bediener Hilfestellungen bei der Zusammenführung und mechanischen Verriegelung der Baueinheiten. Die elektromechanische Schnittstelle hingegen sorgt für die notwendigen elektrischen Kontakte zwischen den Baueinheiten. Der Benutzer muss daher nur die Baueinheiten zusammenkoppeln und erhält dann ohne weitere Maßnahmen unmittelbar eine funktionsfähige Dosiervorrichtung.

Durch die Gestaltung mit Aufnahmeraum können die Führungsmittel, Verriegelungsmittel und empfindliche Teile der elektromechanischen Schnittstelle in einer geschützten Position im Aufnahmeraum untergebracht werden. Der korrespondierende Steckabschnitt kann auf einfache, robuste Geometrien beschränkt werden und ist somit sicherer in der Handhabung durch den Benutzer.

In Ausgestaltung der Erfindung weisen die Führungsmittel zueinander korrespondierende Linearführungsprofilierungen an beiden Baueinheiten auf. Diese beschränken bei der Ankopplung der Baueinheiten einen Bewegungsspielraum zwischen den Baueinheiten auf eine lineare Montagebewegung und sorgen somit für eine korrekte Verbindung der Baueinheiten über die elektromechanische Schnittstelle. Die lineare Montagebewegung ermöglicht eine besonders einfache und bedienerfreundliche An- oder Abkopplung der Baueinheiten.

In weiterer Ausgestaltung der Erfindung sind elektrische Kontaktflächen der elektromechanischen Schnittstelle im Bereich des Aufnahmeraumes und des Steckabschnittes vorgesehen. Die Kontaktflächen erlauben in Wirkverbindung mit korrespondierenden Kontaktzungen eine Übertragung elektrischer Signale zwischen den Baueinheiten nach Einstecken des Steckabschnittes in den Aufnahmeraum, ohne dass dazu besondere Vorkehrungen durch den Benutzer getroffen werden müssen.

In weiterer Ausgestaltung der Erfindung begrenzen der Aufnahmeraum und der Steckabschnitt zumindest abschnittsweise einen Luftkanal für eine definierte Luftzufuhr zu dem Applikator. Der Luftkanal dient zur Bereitstellung eines definierten Luftstroms, der der Umgebung entnommen wird und in der Dosiervorrichtung gemäß den Erfordernissen des Mediumaustrags als laminare oder turbulente Strömung bis in den Applikator geleitet wird. Der Luftstrom wird dabei insbesondere durch eine Einatembewegung des Benutzers hervorgerufen, der einen als Mundstück ausgeführten Applikator mit den Lippen fest umschließt. Der bei der Einatembewegung auftretende Unterdruck bewirkt ein Einströmen von Luft längs der Luftführungseinrichtung durch das Mundstück, insbesondere in den Rachenraum, die Bronchien oder die Lunge des Benutzers. Mit dem definierten Luftstrom kann eine besonders vorteilhafte Dosierung des auszutragenden Mediums vorgenommen werden.

In weiterer Ausgestaltung der Erfindung ist der Applikator zwischen einer Ruheposition und einer Abgabeposition an der Baueinheit beweglich gelagert. In der Ruheposition kann der Applikator durch eine Verschlusseinrichtung vor einer Kontamination durch Mikroben oder Schmutzpartikel geschützt werden. Durch eine Bewegung des Applikators in die Abgabeposition wird eine Applikatoröffnung freigegeben und eine Mediumabgabe kann stattfinden. Durch die bewegliche Lagerung kann der Benutzer eine bequeme und vorteilhafte Ausrichtung des Applikators wählen und somit einen sicheren Mediumaustrag durchführen.

In weiterer Ausgestaltung der Erfindung weist die Steuereinheit Sensormittel zur Erfassung einer Endposition des Applikators auf, wobei die Fördereinrichtung durch die Steuereinheit derartig ansteuerbar ist, dass in der Abgabeposition des Applikators ein Mediumstrom freigegeben und in der Ruheposition des Applikators der Mediumstrom zurückgehalten wird. Durch die Sensormittel, die ein Sensorsignal über eine Positionierung des Applikators an die Steuereinheit übermitteln, kann verhindert werden, dass ein Mediumaustrag erfolgt, wenn der Applikator in der Ruhestellung ist. Insbesondere bei Vorliegen einer Verschlusseinrichtung kann somit eine Fehldosierung verhindert werden, die zu unerwünschten Folgeerscheinungen wie Beschädigungen der Dosiervorrichtung oder Anreicherungen von im Medium enthaltenen Wirkstoffen im Applikator führen könnte.

In weiterer Ausgestaltung der Erfindung sind Arretiermittel zwischen den beiden Baueinheiten vorgesehen, die abhängig von der Stellung des beweglichen Applikators in eine Freigabestellung oder in eine Verriegelungsstellung überführbar sind. Die Arretiermittel verhindern eine ungewollte Entriegelung der Baueinheiten und tragen somit zu einer störungsfreien Funktion der Dosiervorrichtung bei. Um eine Entriegelung der Baueinheiten herbeizuführen, muss der Benutzer den Applikator aus der Verriegelungsstellung, die auch einen größeren Verriegelungsbereich umfassen kann, in eine Freigabestellung überführen. Bei einer vorteilhaften Ausführungsform der Dosiervorrichtung sind für den Applikator Vorspannmittel vorgesehen, die die Aufwendung einer Bedienkraft zur Entriegelung der Baueinheiten voraussetzen.

In weiterer Ausgestaltung der Erfindung weisen die Arretiermittel mechanische Zwangsführungsmittel auf, die die beiden Baueinheiten in den Endpositionen des Applikators miteinander verriegeln und in wenigstens einer Zwischenposition des Applikators die Baueinheiten für eine gegenseitige Trennung freigeben. Damit kann eine einfache konstruktive Gestaltung der Arretiermittel verwirklicht werden, bei der auch eine einfache Bedienung gewährleistet ist. Die Zwangsführungsmittel erlauben eine Trennung der Baueinheiten vorzugsweise lediglich in einer genau definierten Zwischenposition des Applikators, während in allen anderen Positionen des Applikators inklusive der Endpositionen keine Trennung der Baueinheiten möglich ist.

In weiterer Ausgestaltung der Erfindung sind einem Abgabereich des Applikators insbesondere antimikrobielle Wirkmittel zur Keimreduktion zugeordnet. Die Wirkmittel sind zur Vermeidung einer Kontamination und eines Bewuchses von Mediumleitflächen des Applikators vorgesehen und dienen zur Reduzierung damit verbundener Gefahren für einen Benutzer der Dosiervorrichtung. Die Wirkmittel sind insbesondere antimikrobiell ausgeführt und können auf oder in den Mediumleitflächen des Applikators, insbesondere durch Aufbringen oder Einlagern von kupfer- oder silberhaltigen Substanzen, verwirklicht werden. Alternativ können die Wirkmittel auch durch eine Schwammanordnung gebildet sein, die Tropfenreste an der Austrittsöffnung des Applikators aufnimmt und so eine Kontamination verhindern oder zumindest reduzieren kann.

Weitere Vorteile und Merkmale ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in isometrischer Darstellung eine aus zwei Baueinheiten bestehende Dosiervorrichtung,
- Fig. 2: in isometrischer Darstellung einen Ultraschallzerstäuber,
- Fig. 3: in isometrischer Darstellung ein Grundgehäuse.

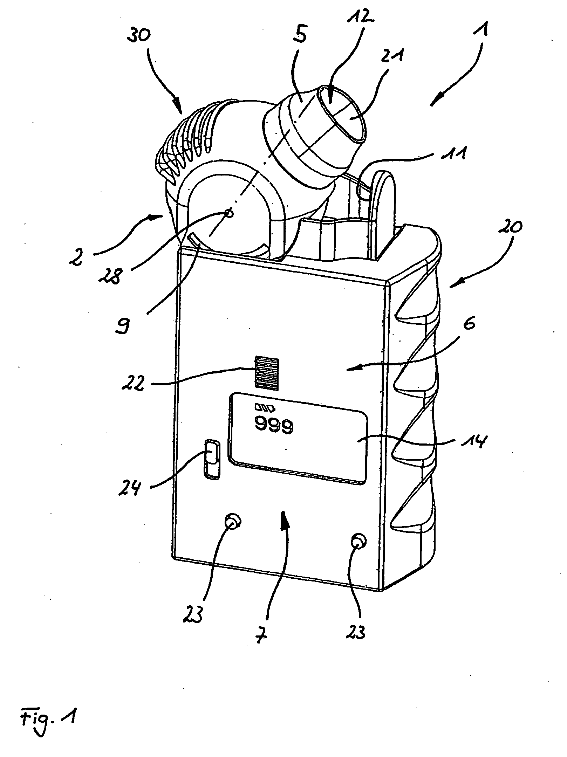

Eine Dosiervorrichtung 1 gemäß der Fig. 1 weist eine als Ultraschallzerstäuber 2 ausgeführte erste Baueinheit auf, die in eine zweite Baueinheit mit einem Grundgehäuse 20 eingesteckt ist.

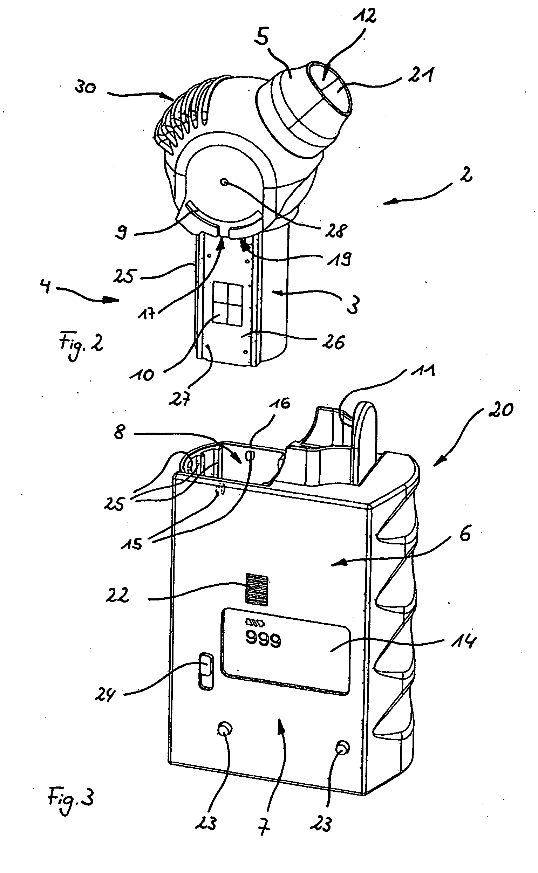

Der in Fig. 2 dargestellte Ultraschallzerstäuber 2 ist mit einem Steckabschnitt 4 versehen, der einen im wesentlichen gleichbleibenden Querschnitt aufweist. An dem Querschnitt sind lineare Führungsschienen 25 angebracht, die für eine Wirkverbindung mit korrespondierenden Führungsschienen 25 des Grundgehäuses 20 vorgesehen sind. Der Steckabschnitt 4 beinhaltet eine nicht dargestellte, elektrisch betriebene Fördereinrichtung und einen nicht näher dargestellten Mediumspeicher 3 und ist mit einem als Mundstück 5 ausgeführten Applikator verbunden. Die Fördereinrichtung kann dabei insbesondere als elektromechanische oder elektronische Mediumpumpe vorgesehen sein, der eine Ultraschallzerstäubung des geförderten Mediums nachgeschaltet sein kann. Der Mediumspeicher 3 kann insbesondere als druckloser Behälter, als zeitweilig druckbeaufschlagter Behälter oder als Druckbehälter mit permanentem Innendruck ausgeführt sein und ist mit dem auszutragenden Medium befüllt. Als Materialien für den Mediumspeicher 3 kommen insbesondere Glas, Kunststoff oder Metall in Frage. Zur zumindest zeitweiligen Erzeugung eines Drucks im Medium können im Mediumspeicher 3 insbesondere mechanische oder pneumatische Druckerzeugungsmittel vorgesehen sein. Als mechanische Druckerzeugungsmittel kommen insbesondere durch äußere Kräfte deformierbare Mediumspeicherabschnitte oder bewegliche Kolben in Frage, die durch mechanische oder elektromechanische Stellmittel mit Stellkräften beaufschlagt werden.

Das Mundstück 5 ist um ein Drehgelenk 28 verschwenkbar an dem Ultraschallzerstäuber 2 angebracht und durch nicht dargestellte Vorspannmittel mit einer Rückstellkraft beaufschlagt. Das Mundstück 5 weist eine Mundstücköffnung 12 auf, durch die ein Gemisch aus auszutragendem Medium und Luft durch einen Benutzer angesaugt werden kann. Das Mundstück 5 weist an einer nach innen gekehrten, als Mundstückwandung 21 ausgeführten Mediumleitfläche eine antimikrobielle Beschichtung auf, die bei längerer Nichtbenutzung der Dosiervorrichtung 1 einen Anlagerung von Mirkoorganismen oder andere Kontaminationen verhindert. Seitlich am Mundstück 5 sind Riegelnuten 9 vorgesehen, die als Kulissenführung für einen im Grundgehäuse 20 vorgesehenen Riegelzapfen 15 dienen können. Durch die Riegelnuten 9 wird ein Formschluss zwischen dem Mundstück 5 und dem Grundgehäuse 20 bei gleichzeitiger Beweglichkeit ermöglicht. Gemäß der Darstellung in der Fig. 1 ist die Dosiervorrichtung 1 bereit für einen Mediumaustrag, wozu das Mundstück 5 durch Aufbringen einer Bedienkraft auf eine Bedienzone 30 aus einer Ruheposition ausgelenkt und um eine durch ein Drehgelenk 28 weisende Drehachse in eine Funktionsstellung geschwenkt wurde.

An einer Seitenfläche des schaftförmig ausgeführten Steckabschnittes 4 des Ultraschallzerstäubers 2 ist eine ebene, flexible Leiterplatte 26 vorgesehen, die elektrische Kontaktflächen 10 aufweist, und die mit der Fördereinrichtung und/oder der Ultraschallzerstäubung verbunden ist. Die Leiterplatte 26 ist mit Sicherungsstiften 27 auf der Oberfläche des Ultraschallzerstäubers 2 fixiert.

Wie aus Fig. 3 entnommen werden kann, sind in dem Grundgehäuse 20 Führungsschienen 25 in einem Aufnahmebereich 8 vorgesehen und dienen zur Führung des Ultraschallzerstäubers 2. Auch weitere, nicht näher dargestellte Komponenten der elektromechanischen Schnittstelle wie federvorgespannte elektrische Kontaktzungen zur Kontaktierung der korrespondierenden elektrischen Kontaktflächen des Ultraschallzerstäubers 2 sind ebenfalls im Aufnahmebereich 8 vorgesehen. Die Führungsschienen 25 ermöglichen die einfache Montage des Steckabschnittes 4 in den Aufnahmeraum 8, wobei nicht dargestellte Klemmeinrichtungen mit Rastnasen zur formschlüssigen Arretierung des Steckabschnittes 4 vorgesehen sind. Dadurch sind über die elektromechanische Schnittstelle sowohl mechanische Halte- und/oder Stellkräfte als auch elektrische und/oder elektronische Signale übertragbar. Die elektrischen Signale sind dabei zur Beeinflussung der Dosiervorrichtung und zur Rückübertragung von Mess- oder Stellwerten an die Steuereinheit vorgesehen.

In dem Grundgehäuse 20 sind außerdem eine nicht näher dargestellte, als Regelelektronik 6 ausgeführte Steuereinheit sowie ein ebenfalls nicht näher dargestellter, als Akkumulator 7 ausgeführter Energiespeicher vorgesehen. Die Regelelektronik 6 ist für eine Ansteuerung der elektrisch betriebenen Fördereinrichtung vorgesehen und kann durch Bedieneinrichtungen aktiviert werden. Zu den Bedieneinrichtungen zählen ein Schiebeschalter 24, mit dem der Ultraschallzerstäuber 2 in seiner in das Grundgehäuse 20 eingesteckten Position ver- und entriegelt wird, und mehrere Drucktasten 23, die für eine Erhöhung oder Reduzierung der Dosiermenge eines Austragvorgangs vorgesehen sind. Weiterhin sind an dem Mundstück 5 nicht dargestellte Sensormittel zur Erfassung von Endpositionen des Mundstücks 5 vorgesehen, die ebenfalls elektrische Signale an die Regelelektronik 6 abgeben können.

Am Grundgehäuse 20 ist ein Lufteinlass 22 vorgesehen, der sich in kanalartiger Form durch das Grundgehäuse 20 hindurchzieht und in Fig.1 in Wirkverbindung mit dem Ultraschallzerstäuber 2 steht. Ein durch den Lufteinlass 22 einströmender Luftstrom wird zwischen dem Steckbereich 4 und dem Aufnahmebereich 8 geführt und über die elektromechanische Schnittstelle in das Mundstück 5 geleitet. Der Luftstrom wird dabei insbesondere durch eine Einatembewegung des Benutzers hervorgerufen, der das Mundstück 5 mit den Lippen fest umschließt und anschließend einatmet. Der bei der Einatembewegung auftretende Unterdruck bewirkt ein Einströmen von Luft in den Lufteinlass 22 durch das Grundgehäuse 20 in das Mundstück 5 und von dort insbesondere in den Rachenraum, die Bronchien oder die Lunge des Benutzers.

Für eine Abdichtung zwischen den Baueinheiten ist am Grundgehäuse 20 eine in Fig. 3 dargestellte, umlaufende, als Gummiprofil 16 ausgeführte Abdichtvorrichtung vorgesehen, die ein unerwünschtes Einströmen von Luft in das Mundstück 5 verhindert. Der Luftstrom kann damit durch die Gestaltung des Lufteinlasses 22 sowie des Aufnahmeraumes 8 und des Steckabschnittes 4 entsprechend den Bedürfnissen des Mediumsaustrags turbulent oder laminar in das Mundstück 5 einströmen.

An dem Grundgehäuse 20 ist eine Verschlusseinrichtung für die Mundstücköffnung 12 des Mundstücks 5 vorgesehen. Das Mundstück 5 dient als Schnittstelle zum Anwender der Dosiervorrichtung 1 und weist Mediumleitflächen 21 für das auszutragende Medium auf. Da es sich bei dem auszutragenden Medium insbesondere um aktive Wirkstoffe handeln kann, muss ein Niederschlag dieser Wirkstoffe auf den Mediumleitflächen in Betracht gezogen werden. Durch den Niederschlag des aktiven Wirkstoffs in Kombination mit Keimen aus einer Umgebung der Dosiervorrichtung 1 kann eine Kontamination der Mediumleitflächen 12 nicht ausgeschlossen werden. Um eine Expositionsdauer der Mediumleitflächen 12 gegenüber der Umgebung möglichst gering zu halten und somit das Risiko der Kontamination zu reduzieren, ist eine Verschlusseinrichtung 11 für die Mundstücköffnung 12 des Mundstücks 5 vorgesehen. Die Verschlusseinrichtung 11 verschließt die Mundstücköffnung 12 kurz nach Beendigung des Mediumaustrags, so dass nur eine endliche, insbesondere unkritische Anzahl von Keimen auf die Mediumleitflächen 12 einwirken können.

Für die Benutzung der Dosiervorrichtung 1 nach dem in den Fig. 1 bis 3 gezeigten Ausführungsbeispiel wird in einem ersten Schritt der Ultraschallzerstäuber 2 mit dem Steckabschnitt 4 in den Aufnahmeraum 8 des Grundgehäuses 20 eingeschoben. Dabei wird der Steckabschnitt 4 durch die Führungsschienen 25 linear geführt. Sobald das Mundstück 5 während des Einschiebevorgangs des Steckabschnittes 4 auf die im Grundgehäuse 20 vorgesehenen Führungszapfen 15 aufläuft, muss das Mundstück in die in Fig. 2 dargestellte Position geschwenkt werden, um ein vollständiges Einschieben in den Aufnahmeraum 8 zu ermöglichen. Dabei gleiten die Führungszapfen 15 in die Riegelnut 9 des Mundstücks 5 und stellen eine formschlüssige Verbindung zwischen den beiden Baueinheiten her. Sofern der Ultraschallzerstäuber 2 korrekt in den Aufnahmeraum 8 des Grundgehäuses 20 eingeschoben ist, kann eine Verriegelung zwischen dem Steckabschnitt 4 und dem Grundgehäuse 20 durch den Schiebeschalter 24 vorgenommen werden. Der Schiebeschalter verriegelt den Steckabschnitt 4 und schaltet gleichzeitig die elektrische/elektronische Funktion der Dosiervorrichtung frei, damit diese in Betrieb genommen werden kann. Dadurch ist eine Sicherungsfunktion verwirklicht, ein Betrieb der Dosiervorrichtung 1 ist nur bei korrekt verriegeltem Ultraschallzerstäuber 2 möglich. Eine Überführung des Schiebeschalters 24 in die entriegelte Position setzt die Dosiervorrichtung und damit den Ultraschallzerstäuber 2 außer Funktion und ermöglicht eine Entnahme des Ultraschallzerstäubers aus dem Grundgehäuse 20.

Durch das Federelement 19 wird das Mundstück 5 in Abwesenheit einer Bedienkraft so verschwenkt, dass die Mundstücköffnung 12 von der Abdeckplatte 11 gegen Kontaminationen aus der Umgebung verschlossen ist. Diese Mundstückposition entspricht der Ruheposition der Dosiervorrichtung. In dieser Ruheposition stellt ein nicht dargestelltes Sensormittel ein Positionssignal an die Regelelektronik 6 zur Verfügung, so dass kein Mediumaustrag erfolgen kann. Das Sensormittel kann als Schalter ausgeführt sein, der ein entsprechendes Schaltsignal an die Regelelektronik 6 oder eine anders geartete Steuereinheit übermittelt.

Durch Verschwenken des Mundstücks 5 aus der Ruheposition wird die Mundstücköffnung 12 freigegeben. Dabei wird durch Zusammenwirken der im Mundstück 5 als Riegelnut 9 ausgeführten Kulissenführung mit Führungszapfen 15 ein Schwenkbereich für das Mundstück 5 begrenzt. Durch die Riegelnut 9 ist zumindest nahezu über den gesamten Schwenkbereich des Mundstücks 5 eine formschlüssige Verriegelung der Riegelnut 9 mit dem Führungszapfen gewährleistet.

Nun kann der Benutzer durch Betätigen einer der Drucktasten 23 die Dosiervorrichtung aktivieren und je nach Bedarf durch weitere Betätigung der Drucktasten 23 Einfluss auf die vorzunehmende Mediumdosierung insbesondere durch die schrittweise Erhöhung oder Reduzierung der auszutragenden Mediummenge nehmen. Anschließend kann der Benutzer das Mundstück 5 mit den Lippen fest umschließen und durch eine Einatembewegung einen Luftstrom durch den Lufteinlass 22 und den nachgeschalteten Luftkanal der Dosiervorrichtung bis in das Mundstück 5 ansaugen. Durch den auftretenden Luftstrom wird eine Messeinrichtung angesprochen, die ein Steuersignal an die Regelelektronik 6 zur Durchführung der Mediumdosierung sendet. Von der Regelelektronik 6 wird dann ein Aktivierungssignal über die Kontaktzungen und die Kontaktflächen 10 auf den Ultraschallzerstäuber 2 übertragen und von dort über die Leiterplatte 26 an die Fördereinrichtung und/oder die Ultraschallkammer weitergeleitet. Die Fördereinrichtung fördert auf das Aktivierungssignal hin eine Mediummenge aus dem Flüssigkeitsbehälter 3. Durch eine nicht dargestellte Ultraschalleinrichtung wird das Medium fein vernebelt und mit dem Luftstrom vermischt. Damit kommt es während der Einatembewegung des Benutzers zu einem Mediumtransport aus dem Flüssigkeitsbehälter 3 in den das Mundstück 5 durchströmenden Luftstrom.

Damit kann das Medium je nach Art der Zerstäubung und nach Größe der aus der Zerstäubung resultierenden Flüssigkeitströpfchen mehr oder weniger weit in den Atemtrakt des Benutzers hineingesaugt werden. Nach Beendigung des Einatemvorganges kann das Mundstück 5 wieder in die Ruheposition zurückgeführt werden und wird durch die Abdeckplatte 11 bis zum nächsten Mediumaustrag verschlossen.

Für eine Auswechslung des Ultraschallzerstäubers 2 aus dem Grundgehäuse 20 wird das Mundstück 5 aus der Ruheposition in die in den Fig. 1 und 2 dargestellte Position verschwenkt und kann dann nach Aufhebung des Formschlusses zwischen der Riegelnut 9 und dem Führungszapfen 15 aus dem Grundgehäuse entnommen werden. Beim Entnehmen des Ultraschallzerstäubers 2 wird auch automatisch der elektrische Kontakt zwischen den federvorgespannten Kontaktzungen im Aufnahmeraum 8 und den Kontaktflächen 10 aufgehoben.

## Patentansprüche

1. Dosiervorrichtung (1) mit einer elektrisch angetriebenen Fördereinrichtung (4) für wenigstens ein Medium, einem Mediumspeicher (3) zur Bevorratung des Mediums und einem Applikator (5) zur Abgabe des Mediums, mit einer Steuereinheit (6) zur Beeinflussung der Fördereinrichtung (4) und mit einem elektrischen Energiespeicher (7) zur Versorgung der Steuereinheit (6) und/oder der Fördereinrichtung (4), wobei die Fördereinrichtung (4), der Mediumspeicher (3) und der Applikator (5) einerseits und die Steuereinheit (6) und der elektrische Energiespeicher (7) andererseits (5) in jeweils separaten Baueinheiten (2, 20) untergebracht sind, und wobei Führungsmittel (25) zum Ineinanderstecken der Baueinheiten (2, 20) vorgesehen sind, die eine elektromechanische Schnittstelle für eine werkzeuglose An- und/oder Abkopplung relativ zu der Steuereinheit (6) und/oder dem Energiespeicher (7) aufweisen,
**dadurch gekennzeichnet, dass**
die eine Baueinheit (20) mit einem zu einer Stirnseite offenen Aufnahmeraum (8) versehen ist, in den ein korrespondierender Steckabschnitt (4) der anderen Baueinheit (2) einführbar ist, wobei der Steckabschnitt den Medienspeicher (3) umfasst.

2. Dosiervorrichtung nach Anspruch 1, **dadurch gekennzeichnet, dass** die Führungsmittel (25) zueinander korrespondierende Linearführungsprofilierungen an beiden Baueinheiten (2, 20) aufweisen.

3. Dosiervorrichtung nach Anspruch 1 oder 2, **dadurch gekennzeichnet, dass** elektrische Kontaktflächen (10) der elektromechanischen Schnittstelle im Bereich des Aufnahmeraumes (8) und des Steckabschnittes (4) vorgesehen sind.

4. Dosiervorrichtung nach einem der vorstehenden Ansprüche, **dadurch gekennzeichnet, dass** der Aufnahmeraum (8) und der Steckabschnitt (4) zumindest abschnittsweise einen Luftkanal (17, 22) für eine definierte Luftzufuhr zu dem Applikator (5) begrenzen.

5. Dosiervorrichtung nach Anspruch 1, **dadurch gekennzeichnet, dass** der Applikator (5) zwischen einer Ruheposition und einer Abgabeposition an der Baueinheit (2) beweglich gelagert ist.

6. Dosiervorrichtung nach einem der vorstehenden Ansprüche, **dadurch gekennzeichnet, dass** die Steuereinheit (6) Sensormittel zur Erfassung einer Endposition des Applikators (5) aufweist, und dass die Fördereinrichtung durch die Steuereinheit (6) derartig ansteuerbar ist, dass in der Abgabeposition des Applikators (5) ein Mediumstrom freigegeben und in der Ruheposition des Applikators (5) der Mediumstrom zurückgehalten wird.

7. Dosiervorrichtung nach einem der vorstehenden Ansprüche, **dadurch gekennzeichnet, dass** Arretiermittel (9, 15) zwischen den beiden Baueinheiten (2, 20) vorgesehen sind, die abhängig von der Stellung des beweglichen Applikators (5) in eine Freigabestellung oder in eine Verriegelungsstellung überführbar sind.

8. Dosiervorrichtung nach Anspruch 7, **dadurch gekennzeichnet, dass** die Arretiermittel (9, 15) mechanische Zwangsführungsmittel aufweisen, die die beiden Baueinheiten (2, 20) in den Endpositioneh des Applikators (5) miteinander verriegeln und in wenigstens einer Zwischenposition des Applikators (5) die Baueinheiten (2, 20) für eine gegenseitige Trennung freigeben.

9. Dosiervorrichtung nach einem der vorstehenden Ansprüche, **dadurch gekennzeichnet, dass** die Dosiervorrichtung in einem Abgabereich (21) des Applikators (5) antimikrobielle Wirkmittel zur Keimreduktion aufweist.

## Claims

1. Dosing device (1) for fluids having an electrically powered conveying device (4) for at least one medium, a medium reservoir (3) for storage of the medium and an applicator (5) for dispensing the medium, having a control unit (6) for influencing the conveying device (4) and having an electrical energy reservoir (7) for supplying the control unit (6) and/or the conveying device (4), where the conveying device (4), the medium reservoir (3) and the applicator (5) on the one hand and the control unit (6) and the electrical energy reservoir (7) on the other hand are accommodated in separate structural units (2, 20), and where guide means (25) are provided for fitting together the structural units (2, 20), which have an electromechanical interface for a tool-free connection and/or disconnection relative to the control unit (6) and/or the energy reservoir (7), **characterized in that**
the one structural unit (20) is provided with a receptacle area (8) open to one end and into which a corresponding insert section (4) of the other structural unit (2) can be inserted, said insert section comprising the medium reservoir (3).

2. Dosing device according to Claim 1, **characterized in that** the guide means (25) have linear guide profiles corresponding to one another on both structural units (2, 20).

3. Dosing device according to Claim 1 or 2, **characterized in that** electrical contact surfaces (10) of the electromechanical interface are provided in the area of the receptacle area (8) and the insert section (4).

4. Dosing device according to one of the preceding claims, **characterized in that** the receptacle area (8) and the insert section (4) limit at least in some sections an air duct (17, 22) for a defined air supply to the applicator (5).

5. Dosing device according to Claim 1, **characterized in that** the applicator (5) is movably mounted on the structural unit (2) between a rest position and a dispensing position.

6. Dosing device according to one of the preceding claims, **characterized in that** the control unit (6) has sensor means for detecting an end position of the applicator (5) and **in that** the conveying device can be controlled by the control unit (6) such that in the dispensing position of the applicator (5) a medium flow is released and in the rest position of the applicator (5) the medium flow is held back.

7. Dosing device according to one of the preceding claims, **characterized in that** locking means (9, 15) are provided between the two structural units which can be transferred to a release setting or to a locking setting depending on the position of the movable applicator (5).

8. Dosing device according to Claim 7, **characterized in that** the locking means (9, 15) have mechanical forcible guide means which interlock the two structural units (2, 20) to one another in the end positions of the applicator (5) and release the structural units (2, 20) for mutual separation in at least one intermediate position of the applicator (5).

9. Dosing device according to one of the preceding claims, **characterized in that** the dosing device has antimicrobial agents for germ reduction in a dispensing area (21) of the applicator (5).

## Revendications

1. Dispositif de dosage (1) avec un dispositif d'alimentation (4) entraîné de manière électrique pour au moins une matière, un réservoir de matière (3) pour l'approvisionnement en matière, et un applicateur (5) pour délivrer la matière, avec une unité de commande (6) permettant d'influencer le dispositif d'alimentation (4) et avec un accumulateur d'énergie (7) électrique pour alimenter l'unité de commande (6) et/ou le dispositif d'alimentation (4), sachant que le dispositif d'alimentation (4), le réservoir de matière (3) et l'applicateur (5) d'une part, et l'unité de commande (6) et l'accumulateur d'énergie (7) électrique d'autre part sont logés dans des unités modulaires (2, 20) respectivement séparées, et sachant que des moyens de guidage (25) sont prévus pour emboîter les unités modulaires (2, 20) qui présentent une interface électromécanique pour une connexion/déconnexion sans outil par rapport à l'unité de commande (6) et/ou l'accumulateur d'énergie (7),
**caractérisé en ce que**
une des unités modulaires (20) est pourvue d'un espace récepteur (8) ouvert sur une face frontale, dans lequel une section enfichable (4) correspondante de l'autre unité modulaire (2) peut être introduite, sachant que la section enfichable comprend le réservoir de matière (3).

2. Dispositif de dosage selon la revendication 1, **caractérisé en ce que** les moyens de guidage (25) présentent des profils de guidage linéaire correspondants les uns aux autres sur les deux unités modulaires (2, 20).

3. Dispositif de dosage selon la revendication 1 ou 2, **caractérisé en ce que** des surfaces de contact électriques (10) de l'interface électromécanique sont prévues dans la zone de l'espace récepteur (8) et de la section enfichable (4).

4. Dispositif de dosage selon l'une des revendications précédentes, **caractérisé en ce que** l'espace récepteur (8) et la section enfichable (4) limitent au moins en partie un canal d'air (17, 22) pour une alimentation en air définie vers l'applicateur (5).

5. Dispositif de dosage selon la revendication 1, **caractérisé en ce que** l'applicateur (5) est logé de manière à pouvoir se déplacer entre une position d'arrêt et une position de distribution sur l'unité modulaire (2).

6. Dispositif de dosage selon l'une des revendications précédentes, **caractérisé en ce que** l'unité de commande (6) présente des moyens capteurs permettant de détecter une position finale de l'applicateur (5), et que l'unité d'alimentation peut être commandée de telle manière par l'unité de commande (6) qu'un flux de matière est libéré en position de distribution de l'applicateur (5) et que le flux de matière est retenu en position d'arrêt de l'applicateur (5).

7. Dispositif de dosage selon l'une des revendications précédentes, **caractérisé en ce que** des moyens d'arrêt (9, 15) sont prévus entre les deux unités modulaires (2, 20) qui, en fonction de la position de l'applicateur (5) mobile, peuvent être amenés dans une position de libération ou dans une position de verrouillage.

8. Dispositif de dosage selon la revendication 7, **caractérisé en ce que** les moyens d'arrêt (9, 15) présentent des moyens mécaniques de guidage forcé qui verrouillent ensemble les deux unités modulaires (2, 20) dans les positions finales de l'applicateur (5) et libèrent, dans au moins une position intermédiaire de l'applicateur (5), les unités modulaires (2, 20) pour une séparation réciproque.

9. Dispositif de dosage selon l'une des revendications précédentes, **caractérisé en ce que** le dispositif de dosage présente dans une zone de distribution (21) de l'applicateur (5) des agents chimiques antimicrobiens afin de réduire les germes.
